# EUROPÄISCHE PATENTSCHRIFT

(11) **EP 1 113 766 B1**
(45) Veröffentlichungstag und Bekanntmachung des Hinweises auf die Patenterteilung: **10.11.2004**
(21) Anmeldenummer: 99948762.2
(22) Anmeldetag: 16.09.1999
(51) Int. Cl.: A61F 2/20

(54) **SET FÜR DEN EINSATZ EINES SHUNT-VENTILS IN EINEN SHUNT ZWISCHEN OESOPHAGUS UND TRACHEA**
SET FOR INSERTING A SHUNT VALVE INTO A SHUNT BETWEEN THE OESOPHAGUS AND THE TRACHEA
ENSEMBLE DESTINE A L'UTILISATION D'UNE VALVE DE DERIVATION DANS UNE CANULE DE DERIVATION PLACEE ENTRE L'OESOPHAGE ET LA TRACHEE

(30) Priorität: 17.09.1998 DE 19842505; 09.11.1998 DE 29819974 U
(43) Veröffentlichungstag der Anmeldung: 11.07.2001
(73) Patentinhaber: Adeva Medical Gesellschaft für Entwicklung und Vertrieb von Medizinischen Implantat-Artikeln mbH, 23556 Lübeck (DE)
(72) Erfinder: SCHUMACHER, Erhard, D-65375 Oestrich-Winkel (DE); SCHULTZ, Thomas, D-21365 Adendorf (DE)
(74) Vertreter: Fuchs Mehler Weiss & Fritzsche
(86) Internationale Anmeldenummer: PCT/EP1999/006853
(87) Internationale Veröffentlichungsnummer: WO 2000/016720

(56) Entgegenhaltungen:
- EP-A- 0 551 198
- WO-A-95/29649
- WO-A-96/35399
- WO-A-97/23341
- US-A- 4 808 183

## Beschreibung

Die Erfindung betrifft ein Set für den Einsatz eines Shunt-Ventils in einem operativ erzeugten Verbindungskanal (Shunt) zwischen Trachea und Oesophagus eines laryngektomierten Patienten.

Der Einsatz von Shunt-Ventilen ist seit langem bekannt. Ein derartiges Shunt-Ventil ist beispielsweise aus der jüngeren deutschen Patentanmeldung 196 51 951 der Anmelderin bekannt. Üblicherweise weisen die Shunt-Ventile zwei umlaufende Flansche auf, von denen nach dem Einsatz in den Verbindungskanal der eine Flansch an der Oesophaguswand und der andere Flansch an der Tracheawand zum Liegen kommt.

Neuere Entwicklungen weisen anstelle des oesophagusseitigen umlaufenden Flansches größere, flexiblere Haltelaschen auf, die sich nach dem Einsatz des Ventils in radialer Richtung quasi entfalten und sich großflächig gegen die Oesophaguswand anlegen, um so für einen sicheren Halt des Ventils im Verbindungskanal zu sorgen.

Problematisch dabei ist die Art und Weise, wie ein Shunt-Ventil mit vergrößerten Haltelaschen überhaupt in den Verbindungskanal gesetzt werden kann, ohne dem Patienten allzuviel Unannehmlichkeiten oder gar Schmerzen zu bereiten. Im Grunde genommen besteht die Hauptschwierigkeit beim Einsatz des Ventils in den Verbindungskanal darin, die flexiblen großen Haltelaschen, die radial von der Hauptachse des Ventils abstehen, in geeigneter Weise zusammenzurollen und sich erst nach dem Einsatz des Ventils entfalten zu lassen.

Da dies von Hand nicht möglich ist, muß ein Instrument oder Halter für das Shunt-Ventil verwendet werden. Ein Vorschlag, die erwähnten Haltelaschen während des Einsetzens im zusammengerollten Zustand zu halten, sieht vor, die Haltelaschen mittels eines lebensmittelechten Klebers in der entsprechenden Lage zu halten. Im Laufe der Zeit nach dem Einsatz des Shunt-Ventils löst sich der Kleber auf und die Haltelaschen entfalten sich in vorgesehener Weise. Der Zeitpunkt der Entfaltung ist jedoch nicht exakt bestimmbar, woraus Probleme erwachsen können.

Aus der WO-A- 95/29649 ist ein Set für den Einsatz eines Shunt-Ventils gemäß dem Oberbegriff des Anspruchs 1 bekannt.

Vor diesem Hintergrund ist es die Aufgabe der vorliegenden Erfindung, ein Komplett-Set für den Einsatz eines Shunt-Ventils anzugeben, mit dem der Einsatz in den operativ erzeugten Shunt problemlos möglich ist und welches den Vorgang des Einsatzes für die behandelnde Person erleichtert.

Gelöst wird diese Aufgabe durch das Set gemäß Anspruch 1. Vorteilhafte Weiterbildungen sind in den Unteransprüchen angegeben.

Demgemäß wird ein Set für den Einsatz eines Shunt-Ventils in einen operativ erzeugten Verbindungskanal zwischen Trachea und Oesophagus eines laryngektomierten Patienten vorgeschlagen, wobei das Shunt-Ventil oesophagusseitig wenigstens zwei flexible, zusammengerollte Haltelaschen aufweist, die sich nach dem Einsatz in dem Verbindungskanal aufrichten lassen und gegen die Oesophaguswand legen, aufweisend einen Halter mit einem proximalen, im wesentlichen zylindrischen Kupplungsstück, auf welches das Shunt-Ventil gesetzt ist, sowie ein Halte- und Auslöseelement, welches die wenigstens zwei Haltelaschen lösbar in zusammengerollter Stellung hält und auf Betätigung hin die Haltelaschen freigibt.

Das erfindungsgemäße Set eignet sich besonders zur Wiederverwendung bzw. zur Nachversorgung eines Patienten mit einem beispielsweise gereinigten Shunt-Ventil in einer Arztpraxis. Hierzu kann der Arzt das Instrument in einfacher Weise mit einem Shunt-Ventil bestücken, die besagten Haltelaschen aufrollen und diese in dieser Lage durch Hindurchführung der Führungsstange mit ihrem balligen Ende durch die Öffnungen in den Haltelaschen halten oder arretieren. Das flexible Material der Haltelaschen wird bei Hindurchführung des balligen Endes der Führungsstange gedehnt, so daß sich die Öffnungen mit geringem Durchmesser in den Haltelaschen beim "Laden" des Instrumentes aufweiten und hinterher wieder zusammenziehen, so daß die zusammengerollten Haltelaschen in ihrer Lage gehalten werden.

Die Auslösung erfolgt bei dieser zweiten Ausführungsform in einfachster Weise durch Ziehen am rückwärtigen Ende der Führungsstange, woraufhin das ballige Ende der Führungsstange die Öffnungen in den Haltelaschen unter elastischer Aufweitung des Materials der Haltelaschen durchtritt und so die arretierende Wirkung des balligen Endes der Führungsstange aufgehoben wird. Das elastische Material des Shunt-Ventils sorgt dafür, daß in diesem Moment sich die Haltelaschen in der vorgesehenen und beschriebenen Weise entfalten.

Die Verwendung des Sets gemäß dieser Ausführungsform durch die behandelnde Person entspricht bis auf den Auslösevorgang der weiter oben beschriebenen Verwendung der ersten Ausführungsform des Sets.

Das Set kann vorteilhaft so ausgebildet werden, daß der Halter vom distalen Griffende längsgestreckt verläuft und zum proximalen Teil hin in einem Bogen gekrümmt ist, um dann in dem Kupplungsstück auszulaufen oder zu enden, wobei die Hauptachse des Kupplungsstückes in einem Winkel zwischen 70 bis 90° zur Längsachse des Griffendes steht. Diese Formgebung des Halters erleichtert die Handhabung des Sets, indem nämlich das proximale Ende, auf welchem das vorbereitete Shunt-Ventil sitzt, durch das Tracheostoma hin zum Verbindungskanal zwischen Trachea und Oesophagus geführt werden kann. Die gekrümmte Form erleichtert gleichwohl die visuelle Kontrolle durch die behandelnde Person hinsichtlich der richtigen Führung und Lage des Ventils.

Die vorerwähnte Weiterbildung kann noch besonders bevorzugt dadurch weitergebildet werden, daß die Führungsstange rückseitig aus dem genannten Bogen austritt und an einem Betätigungsbügel angelenkt ist, der seinerseits am Halter schwenkbar gehaltert ist.

Diese einfache Mechanik erlaubt eine mühelose Handhabung des Sets durch den behandelnen Arzt. Im "geladenen" Zustand des Sets mit aufgesetztem Shunt-Ventil mit zusammengerollten Haltelaschen ist die Führungsstange in ihre maximale Verschiebeposition gedrückt. Die Schwenkhalterung des Betätigungsbügels ist nun so ausgeführt, daß der behandelnde Arzt beispielsweise auf dessen Ende drückt, woraufhin der Betätigungsbügel verschwenkt wird, derart, daß die Führungsstange aus dieser Maximalposition zurückgezogen wird, wobei das proximale ballige Ende - wie beschrieben - unter Aufweitung des Materials die Öffnungen in den Haltelaschen durchtritt, woraufhin sich die Haltelaschen entfalten.

Die Erfindung wird anhand eines Ausführungsbeispiele näher erläutert. Hierbei zeigt:
- Figur 1: in Seitenansicht die Hauptkomponenten Shunt-Ventil und Halter des Sets gemäß einer ersten Ausführungsform vor dem Zusammenbau,
- Figur 2: das Set im einsatzbereiten Zustand,
- Figur 3: das proximale Ende des Sets aus Figur 2,
- Figur 4: die rückseitige Ansicht des Halters,
- Figur 5: die Seitenansicht des Sets anderer Ausführungsform im einsatzbereiten Zustand, und
- Figur 6: das Set gemäß Fig. 5 nach Auslösung.

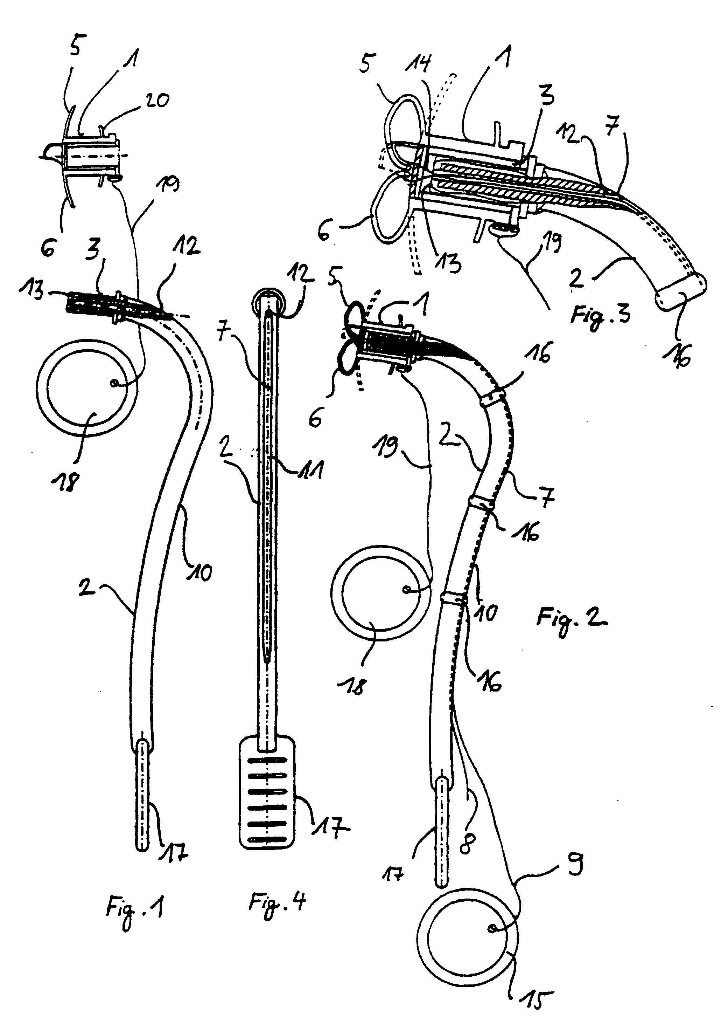

In allen Zeichnungsfiguren sind gleiche Teile mit denselben Bezugszeichen versehen. Die Figuren 1-4 zeigen ein bekanntes Set.

In Figur 1 ist der Halter 2 des Sets in einer geschwungenen Form dargestellt. Hierbei verläuft der Halter 2 vom distalen Griffende 17 her längsgestreckt in einen Bogen in sein proximales Ende aus, welches gebildet ist durch das Kupplungsstück 3 mit seiner Stirnseite 13.

Auf das Kupplungsstück 3 kann das Shunt-Ventil 1 geschoben werden. Das Shunt-Ventil weist einen umlaufenden Flansch 20 und zwei radial abstehende flexible Haltelaschen 5 und 6 auf. An das Shunt-Ventil 1 ist über ein Band 19 ein Ring 18 angebunden, mittels dessen das Ventil 1 bei Bedarf aus dem Verbindungskanal zwischen Trachea und Oesophagus gezogen werden kann.

Das Kupplungsstück 3 ist durch eine Durchbohrung 12 durchsetzt, welche sich im Bereich des geschwungenen Bogens des Halters 2 öffnet. Beim Zusammenbau des Sets wird nun das Shunt-Ventil 1 auf das Kupplungsstück 3 gesetzt. Allerdings muß zunächst der Zugfaden 7 in die rückseitige Nut 11 im Rücken 10 des Halters eingelegt werden, so daß das eine freie Ende 8 des Zugfadens 7 im Bereich des Griffendes 17 endet und der Faden 7 in der Nut 11 geführt durch die Durchbohrung 12 gesetzt wird. Aus der Stirnseite 13 des Kupplungsstückes 3 tritt der Zugfaden 7 aus und bildet hier eine Schlinge 14 unter Durchsetzung der beiden Haltelaschen 5 und 6 (Figur 3). Hierzu ist in jeder Haltelasche 5 und 6 ein Loch eingebracht, durch welches hindurch der Zugfaden 7 geführt wird. Der Zugfaden wird dann zurück in die Durchbohrung 12 gesetzt und den Halter 2 herabgerührt (Figur 2). Dort ist sein zweites freies Ende 9 mit einem Zugring 15 versehen, der das Herausziehen des Zugfadens 7 aus dem Halter 2 erleichtert.

Die Führung des Zugfadens 7 in der Nut 11 des Halters 2 wird vorliegend stabilisiert durch den Halter 2 umgreifende Halterungen 16, die beispielsweise durch Gummiringe gebildet sein können.

Auf diese Weise ist es möglich, daß der Zugfaden 7 die zusammengerollten Haltelaschen 5 und 6 in diesem Zustand halten.

In Figur 3 sind im Übrigen gestrichelt dargestellt die Haltelaschen 5 und 6 nach dem Entfalten, so daß sie sich in diesem Zustand an die Oesophaguswand anlegen können. Auslöser für die Entfaltung ist - wie erläutert - das Ziehen an dem Zugfaden 7 bis die Schlinge 14 geöffnet wird.

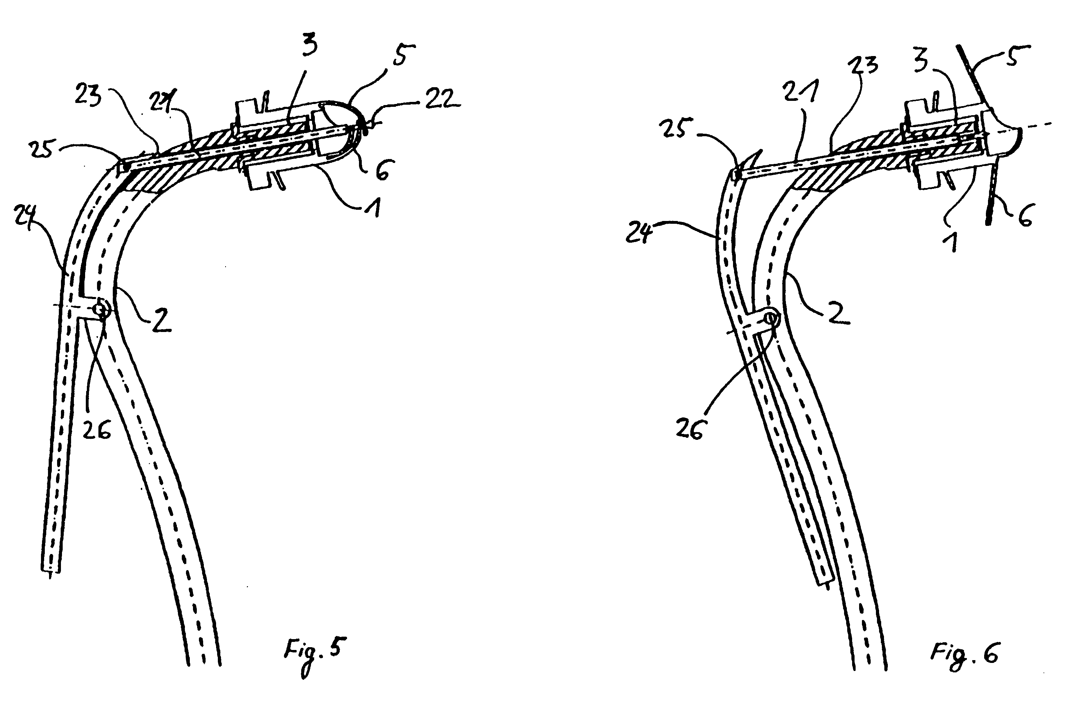

Die Figuren 5 und 6 zeigen eine andere Ausführungsform des Sets. Im folgenden wird lediglich auf die Unterschiede zu dem Set gemäß der ersten Ausführungsform, wie oben beschrieben, hingewiesen.

Figur 5 zeigt das Set dieser Ausführungsform im "geladenen" Zustand mit dem auf dem zylindrischen Kupplungsstück 3 befindlichen Shunt-Ventil 1. Die Haltelaschen 5 und 6 sind vorliegend zusammengefaltet oder aufgerollt und werden vorliegend in diesem Zustand gehalten durch die Führugnsstange 21. Die Führungsstange 21 weist proximal ein balliges Ende 22 auf, welches die zusammengefalteten Haltelaschen 5 und 6 festhält. Das Material der Haltelaschen 5 und 6 ist flexibel, so daß das ballige Ende 22 der Führungsstange 21 unter Aufweitung von entsprechend in den Haltelaschen vorgesehenen Öffnungen durch diese geschoben werden kann.

Die Führungsstange 21 verläuft durch das zylindrische Kupplungsstück 3 hindurch und ist dort verschieblich gelagert.

Im Bereich des Bogens 23 tritt die Führungsstange 21 rückseitig aus dem Halter 2 heraus. An ihrem distalen Ende ist die Führungsstange 21 mittels eines beispielsweise Stiftes 25 an einen Betätigungsbügel 24 angelenkt, der seinerseits am Halter 2 bei 26 schwenkbar angelenkt ist.

Das Set nach Auslösung des Betätigungsbügels 24 zeigt Figur 6. Der behandelnde Arzt hat den Betätigungsbügel 24 in Richtung auf den Halter 2 gedrückt, so daß das Vorderende des Betätigungsbügels 24 eine entsprechende Schwenkbewegung um 26 ausführt. Dabei nimmt der Betätigungsbügel 24 die bei 25 angelenkte Führungsstange 21 mit, d.h., die Führungsstange 21 wird um eine gewisse Strecke nach außen gezogen. Hierbei durchtritt das ballige Ende 22 der Führungsstange 21 die Öffnungen in den Haltelaschen 5 und 6, wieder unter leichter Aufweitung deren Durchmessers. Sobald das proximale Ende 22 der Führungsstange 21 durch die Haltelaschen 5 und 6 hindurchgetreten ist, falten sich die Haltelaschen 5 und 6 - wie in Figur 6 dargestellt - aufgrund der wirkenden Rückstellkräfte auf.

Die zweite Ausführungsform läßt sich in einfacher Weise von dem behandelnden Arzt wiederverwenden durch einfaches Aufschieben eines Shunt-Ventils 1 auf das zylindrische Kupplungsstück 3 und daran anschließendes Zusammenfalten der Haltelaschen 5 und 6 und Hindurchschieben des proximalen Endes 22 durch die Öffnungen in den Haltelaschen 5 und 6.

## Patentansprüche

1. Set für den Einsatz eines Shunt-Ventils (1) in einen operativ erzeugten Verbindungskanal zwischen Trachea und Oesophagus eines laryngektomierten Patienten, aufweisend ein Shunt-Ventil (1), das oesophagusseitig wenigstens zwei flexible, zusammengerollte Haltelaschen (5,6) aufweist, die nach dem Einsatz in den Verbindungskanal aufrichtbar sind und gegen die Oesophaguswand legbar sind, einen Halter (2) mit einem proximalen, im wesentlichen zylindrischen Kupplungsstück (3), auf welches das Shunt-Ventil (1) gesetzt ist, sowie ein Halte- und Auslöseelement (21), welches die wenigstens zwei Haltelaschen (5, 6) lösbar in zusammengerollter Stellung hält und auf Betätigung hin die Haltelaschen (5, 6) freigibt, **dadurch gekennzeichnet, daß** das Halte- und Auslöseelement (21) eine das im wesentlichen zylindrische Kupplungsstück (3) durchdringende, darin verschieblich geführte Führungsstange (21) mit einem balligen proximalen Ende (22) ist, welches durch Öffnungen in den zusammengerollten Haltelaschen (5, 6) setzbar ist und diese an einem Auffalten ohne Abziehen der Führungsstange (21) hindert.

2. Set nach Anspruch 1, bei dem der Halter (2) vom distalen Griffende (17) längsgestreckt verläuft und zum proximalen Teil hin in einem Bogen gekrümmt ist, um dann in dem Kupplungsstück (3) auszulaufen, dessen Hauptachse in einem Winkel zwischen 70 bis 90° zur Längsachse des Griffendes (17) steht.

3. Set nach Anspruch 2, bei dem die Führungsstange (21) rückseitig aus dem Bogen (23) austritt und an einen Betätigungsbügel (24) angelenkt ist, der seinerseits am Halter (2) schwenkbar gehaltert ist.

## Claims

1. Set for the use of a shunt valve (1) in a surgically produced connection channel between trachea and oesophagus of a laryngectomied patient, having a shunt valve (1), which has on the oesophagus side at least two flexible, rolled-together holding flaps (5, 6), which can be raised after use in the connection channel and can be placed against the oesophagus wall, a holder (2) with a proximal, essentially cylindrical coupling piece (3), on which the shunt valve (1) is placed, and a hold and release element (21), which holds the at least two holding flaps (5, 6) releasably in rolled-together position and releases the holding flaps (5, 6) on actuation, **characterised in that** the hold and release element (21) is a guide rod (21) penetrating the essentially cylindrical coupling piece (3) and guided displaceably in the latter and having a spherical proximal end (22), which can be placed through openings in the rolled-together holding flaps (5, 6) and prevents them from unfolding without withdrawing the guide rod (21).

2. Set according to claim 1, in which the holder (2) extends longitudinally from the distal gripping end (17) and is curved in an arc towards the proximal part to then run out in the coupling piece (3), the main axis of which is at an angle between 70 to 90° to the longitudinal axis of the gripping end (17).

3. Set according to claim 2, in which the guide rod (21) emerges from the arc (23) at the rear side and is pivoted at an actuation handle (24), which in turn is held pivotably on the holder (2).

## Revendications

1. Ensemble pour l'insertion d'une prothèse vocale (1) dans un canal de raccordement créé de façon opératoire entre la trachée et l'oesophage d'un patient ayant subi une laryngectomie, comprenant une prothèse vocale (1), qui comprend du côté de l'oesophage au moins deux pattes de retenue (5, 6) flexibles, enroulées, qui, après l'insertion, peuvent être redressées dans le canal de raccordement et qui peuvent être placées contre la paroi de l'oesophage, un support (2) avec une pièce d'accouplement (3) proximale, sensiblement cylindrique, sur laquelle est posée la prothèse vocale (1), ainsi qu'un élément de retenue et de déclenchement (21), lequel retient de manière desserrable l'une des deux pattes de retenue (5, 6) dans la position enroulée et libère les pattes de retenue (5, 6) par suite d'un actionnement, **caractérisé en ce que** l'élément de retenue et de déclenchement (21) est une tige de guidage (21) traversant la pièce d'accouplement (3) sensiblement cylindrique, guidée de manière mobile à l'intérieur de ladite pièce, avec une extrémité proximale convexe (22), laquelle peut être montée à travers des ouvertures dans les pattes de retenue (5, 6) enroulées et qui empêche un dépliement sans retrait de la tige de guidage (21).

2. Ensemble selon la revendication 1, dans lequel le support (2) s'étend longitudinalement depuis l'extrémité de saisie distale (17) et est recourbé du côté de la partie proximale selon un arc, afin de sortir ensuite dans la pièce d'accouplement (3), dont l'axe principal se situe dans un angle compris entre 70 et 90° par rapport à l'axe longitudinal de l'extrémité de saisie (17).

3. Ensemble selon la revendication 2, dans lequel la tige de guidage (21) sort côté arrière de l'arc (23) et est articulée sur un étrier d'actionnement (24), qui est soutenu pour sa part de manière pivotante sur le support (2).
